# EUROPÄISCHE PATENTSCHRIFT

(11) **EP 0 296 341 B1**
(45) Veröffentlichungstag und Bekanntmachung des Hinweises auf die Patenterteilung: **17.03.1993**
(21) Anmeldenummer: 88107269.8
(22) Anmeldetag: 06.05.1988
(51) Int. Cl.: C07C 43/13, C07C 41/16, C07C 31/42, C07C 29/62

(54) **Verfahren zur Herstellung von Polyglycerinen**
Process for the preparation of polyglycerines
Procédé de préparation de polyglycérines

(30) Priorität: 25.06.1987 DE 3721003; 08.04.1988 DE 3811826
(43) Veröffentlichungstag der Anmeldung: 28.12.1988
(73) Patentinhaber: DEUTSCHE SOLVAY-WERKE GMBH, 42697 Solingen (DE)
(72) Erfinder: Jakobson, Gerald, Dr. Dipl.-Chem., D-4134 Rheinberg (DE); Linke, Horst, D-4134 Rheinberg (DE); Siemanowski, Werner, Dr. Dipl.-Chem., D-4134 Rheinberg (DE)
(74) Vertreter: Seiler, Siegfried

(56) Entgegenhaltungen:
- DE-A- 2 455 327
- DE-A- 3 410 520
- DE-C- 197 308
- DE-C- 197 309
- US-A- 2 520 670
- CHEMICAL ABSTRACTS, Band 7, Nr. 18, 20. September 1913, Seite 3113, Columbus, Ohio, US; J. NIVIERE: "Action of alpha-monochlorohydrin and epichlorohydrin on monosodium glycerate"

## Beschreibung

Die Vorliegende Erfindung betrifft ein Verfahren zur Herstellung von Polyglycerinen durch Umsetzung von Glycerinen in der Wärme.

Nach der Literaturstelle "Chemical Abstracts" 7 (18), Seite 3113 (D2) wird α-Monochlorhydrin, das aus Glycerin mit wasserfreiem Chlorwasserstoff bei 120 bis 130 °C hergestellt wird, mit Mono- Natriumglycerinolat zu Glycidol und 2 bis 3 % Diglycerin sowie Verunreinigungen umgesetzt. Das α-Monochlorhydrin dient dabei lediglich zur Herstellung von stark verunreinigten Glycidol, also einer chemisch anderen Verbindung.

Aus der DE-B-197308 (D3) und DE-B-197309 (D4) ist die Herstellung von Mono- und Dichlorhydrin aus Glycerin und gasförmiger Salzsäure (D3) oder Salzsäure (D4) in Gegenwart einer organischen Säure bekannt.

Nach dem Verfahren gemäß US-A-2520670 wird α-Monochlorhydrin des Glycerins in Gegenwart von konzentrierten wässrigen Alkalihydroxidlösungen bei einer erhöhten Temperatur umgesetzt und die Reaktionsmischung mit einem niederen aliphatischen Alkohol (z.B. Methylalkohol, nach Beispielen 1 und 2) behandelt, um die anorganischen Bestandteile auszufällen und nachfolgend filtriert.

Die abgetrennten anorganischen Bestandteile stellen dabei ein Gemisch von anorganischen Salzen, Diglycerin, Alkohol und anderen organischen Verbindungen oder organischen Verunreinigungen dar und lassen sich schwer aufarbeiten.

Das von den ausgefallenen Salzen abgetrennte Reaktionsmedium, das den überwiegenden Anteil an aliphatischen Alkohol, Diglycerin, Polyglycerin, Glycerin, organischen Verunreinigungen und dergleichen enthält, wird mit Salzsäure neutralisiert (wobei sich zusätzlich anorganische Salze bilden) und konzentriert bzw. destilliert.

Die destillative Auftrennung des Rohrprodukts in Glycerin, Diglycerin und Polyglycerin erweist sich insofern schwierig, als im Rohprodukt erhebliche Anteile an Alkalichlorid, die aufgrund der nachträglich erfolgten Neutralisation gebildet werden bzw. sich noch gelöst im Rohprodukt befanden, zugegen sind und Crackprozesse bei der Destillation (es sind sehr hohe Temperaturen erforderlich) initiieren. Die Produkte sind somit verunreinigt, das Verfahren ist großtechnisch in dieser Art nicht durchführbar.

Aus der DE-OS 24 55 327 ist bereits ein Verfahren zur Herstellung von Polyglycerinen (gegebenenfalls mit geringem Anteil an cyclischen Komponenten) durch sauer katalysierte Umsetzung von Glycerinen in der Wärme bekannt, wobei das Glycerin in Gegenwart von Schwefelsäure und Glycerinacetat unterhalb von 2660 Pa (20 mm Hg) zum Polyglycerin kondensiert wird. Nachteilig ist bei diesem Verfahren die Bildung von Nebenprodukten wie Acrolein und Schwefelsäureester. Ein weiterer Nachteil besteht in der notwendigen Verwendung von absolut reinem Glycerin, da sonst eine starke Verfärbung der Ansätze durch die heiße Schwefelsäure erfolgt.

Ziel und Aufgabe der vorliegenden Erfindung war es, diese Nachteile zu vermeiden und ein Verfahren zu finden, das unter geringem apparativen Aufwand sowie ohne weitere Zusätze von Hilfsmitteln die Herstellung von Polglycerinen ermöglicht.

Erfindungsgemäß wurde festgestellt, daß diesen Zielen und Aufgaben ein Verfahren zur Herstellung von Polyglycerinen (gegebenenfalls mit geringem Anteil an cyclischen Komponenten) durch Umsetzung von Glycerinen in der Wärme gerecht wird, wobei Glycerin und/oder Diglycerin bei Temperaturen von 340 K bis 410 K mit gasförmigem Chlorwasserstoff oder mit konzentrierter Salzsäure in Gegenwart eines Katalysators zu einem Gemisch, bestehend im wesentlichen aus α-Monochlorhydrin des Glycerins oder des Diglycerins und Glycerin und/ oder Diglycerin umgesetzt und das entstandene Gemisch oder das aus dem Gemisch abgetrennte α-Monochlorhydrin des Glycerins oder Diglycerins bei Temperaturen von 320 K bis 410 K zu Alkaliglycerinolat und/oder Alkalidiglycerinolat gegeben wird, wonach das so hergestellte Glycerin- und/oder Diglycerin-Polyglyceringemisch von den entstandenen Alkalichloriden getrennt, das Glycerin- und/oder Diglycerin-Polyglyceringemisch durch Wasserzugabe auf eine 70 bis 40 Gew.-%ige Lösung verdünnt und bei Temperaturen von 30 bis 80 °C über eine Kombination von stark sauren Kationen- und nachfolgenden schwach basischen Anionenaustauschern geleitet und entsalzt sowie nachfolgend das nicht umgesetzte Glycerin und gegebenenfalls ein Teil des Diglycerins durch fraktionierte Destillation von dem Polyglycerin, das arm an cyclischen Komponenten ist, getrennt wird.

Die Umsetzung des Glycerins und/oder Diglycerins mit gasförmigem Chlorwasserstoff oder mit konzentrierter Salzsäure erfolgt dabei vorzugsweise in Gegenwart einer flüssigen gesättigten C₁-C₃ Carbonsäure, in einer Konzentration des Katalysators (bezogen auf die Gesamtgewichtsmenge des eingesetzten Glycerins und/oder Diglycerins von 0,05 bis 3 Gew.-%, vorzugsweise 0,5 bis 2 Gew.-%.

Gemäß einer bevorzugten Ausführungsform des erfindungsgemäßen Verfahrens wird das Glycerin und/oder Diglycerin bei Temperaturen von 340 K bis 410 K mit gasförmigem Chlorwasserstoff oder mit konzentrierter Salzsäure in Gegenwart eines Katalysators umgesetzt und das entstandene Gemisch oder das aus dem Gemisch abgetrennte α-Monochlorhydrin des Glycerins oder Diglycerins bei Temperaturen von 320 K bis 410 K zu einem Alkaliglycerinolat und/oder Alkalidiglycerinolat, das eine alkalisch rea-gierende Substanz, vorzugsweise Alkalihydroxid, enthält, gegeben, wonach das so hergestellte Glycerin- und/oder Diglycerin-Polyglyceringemisch von den entstandenen Alkalichloriden sowie das Glycerin und/oder Diglycerin von dem Polyglycerin getrennt werden.

Nach einer bevorzugten Ausführungsform weist das mit gasförmigem Chlorwasserstoff oder mit konzentrierter Salzsäure umzusetzende Glycerin und/oder Diglycerin einen Glycerin- und/oder Diglyceringehalt von mehr als 80 Gew.-%, vorzugsweise mehr als 90 Gew.-%, auf. Das nicht umgesetzte Glycerin sowie gegebenenfalls ein Teil des Diglycerins werden vorzugsweise durch fraktionierte Destillation von dem Polyglycerin getrennt.

Das eingesetzte Alkaliglycerinolat und/oder Alkalidiglycerinolat weist einen Alkaliglycerinolat- oder Alkalidiglycerinolatgehalt von mehr als 80 Gew.-%, vorzugsweise mehr als 90 Gew.-%, auf und enthält vorzugsweise zusätzlich einen alkalisch reagierenden Bestandteil, vorzugsweise Alkalihydroxid.

Als Chlorwasserstoffgas wird im Rahmen des Verfahrens reiner Chlorwasserstoff oder ein Chlorwasserstoffgasgemisch, vorzugsweise ein Chlorwasserstoffgasgemisch, das bei der Vinylchloridherstellung und/oder Allylchloridherstellung anfällt, eingesetzt.

Als konzentrierte Salzsäure wird eine Salzsäure eingesetzt, die ein Gehalt von 10 bis 40 Gew.-%, vorzugsweise 30 bis 35 Gew.-%, HCl aufweist.

Nach einer bevorzugten Ausführungsform des erfindungsgemäßen Verfahrens wird das α-Monochlorhydrin des Glycerins und/oder Diglycerins in das vorgelegte Glycerinolat und/oder Diglycerinolat unter Umrühren zugefügt, vorzugsweise durch kontinuierliche Zugabe.

Gemäß einer vorteilhaften Ausführungsform des erfindungsgemäßen Verfahrens wird das α-Monochlorhydrin des Glycerins und/oder Diglycerins gleichzeitig mit dem Glycerinolat und/oder Diglycerinolat kontinuierlich in einen vorzugsweise thermostabilisierten Durchlaufreaktor eingetragen, wodurch kurze Reaktionszeiten erzielt werden können.

Nach vollendeter Reaktion oder nach der Bildung der Polyglycerine wird vorzugsweise ein Teil des entstandenen Alkalichlorids durch an sich bekannte physikalische Verfahren, vorzugsweise durch Sedimentation und/oder Zentrifugation als Feststoff und der andere Teil nach Zugabe von Wasser über Ionenaustauscher in an sich bekannter Weise vom entstandenen Polyglyceringemisch, beispielsweise gemäß dem Verfahren der DE-OS 34 10 520, abgetrennt.

Weiterhin sollte eine verbesserte Reinigung des erfindungsgemäß erhaltenen Glycerin- und/oder Diglycerin-Polyglyceringemisches erzielt werden.

Erfindungsgemäß erfolgt die Reinigung des erhaltenen Glycerin- und/oder Diglycerin-Polyglyceringemisches durch Verdünnung mit Wasser auf eine 70 bis 40 gew.-%ige Lösung, vorzugsweise 60 bis 50 gew-%ige Lösung, die bei Temperaturen von 30 bis 80°C, vorzugsweise 40 bis 60°C über eine Kombination von stark sauren Kationen- und nachfolgenden schwach basischen Anionenaustauschern entsalzt wird.

Die Regenerierung der Kationenaustauschermasse in den Kationenaustauschern erfolgt bevorzugt mittels einer Gleichstrom- oder Verbund-Gleichstrom-Regenerierung.

Nach einer bevorzugten Ausführungsform werden nach der Regenerierung die Salze ausgewaschen. Nach Beendigung des Auswaschvorganges wird die polyglycerinhaltige, vorzugsweise diglycerinhaltige Lösung durch die Ionenaustauscher durchgeleitet und die den Anionenaustauscher verlassende polyglycerinhaltige, vorzugsweise diglycerinhaltige Lösung bis zur Erreichung eines Polyglyceringehaltes, vorzugsweise Diglyceringehaltes, von 20 Gew.-%, vorzugsweise bis zur Erreichung eines Polyglyceringehaltes, vorzugsweise Diglyceringehaltes, von 15 Gew.-%, zurückgeleitet und zur Herstellung der 70 - 40 gew.-%igen, vorzugsweise 60 - 50 gew.-%igen, polyglycerinhaltigen, vorzugsweise diglycerinhaltigen Ausgangslösung mitverwendet.

Die Durchleitung der polyglycerinhaltigen, vorzugsweise diglycerinhaltigen Lösung erfolgt durch die Ionenaustauscher bevorzugt unter einem Überdruck.

Die polyglycerinhaltige, vorzugsweise diglycerinhaltige Lösung wird dabei unter einem Druck von 1,1 - 10 bar, vorzugsweise 2 - 6 bar, durch die Ionenaustauscher, d.h. durch einen oder mehreren Kationenaustauscher und mindestens einen Anionenaustauscher, geleitet. Zur Steuerung und Aufrechterhaltung des Druckes sind an einer oder mehreren Stellen in der Leitung oder an den Ionenaustauschern Ventile angebracht.

Dabei wird zweckmäßig die polyglycerinhaltige, vorzugsweise diglycerinhaltige Lösung mit einer Strömungsgeschwindigkeit von 0,5 m/h bis 15 m/h, vorzugsweise 1 m/h bis 5 m/h, durch die Ionenaustauscher geleitet.

Als Kationenaustauschermassen und Anionenaustauschermassen werden bevorzugt solche verwendet, die temperaturbeständig bis über 80°C, vorzugsweise bis über 100°C, sind.

Die Ionenaustauschermasse des Kationenaustauschers und/oder Anionenaustauschers ist zweckmäßig von einer Siebplatte, Lochplatte oder einer in der Höhenrichtung des Ionenaustauschers verschiebbar angeordneten, die Austauschermasse abdeckenden und einen gleichmäßigen Flüssigkeitsdurchtritt ermöglichenden Vorrichtung und/oder einer inerten Preßmasse und/oder elastischen Kunststoffmasse bedeckt.

Die stark saure Kationenaustauschermasse und die schwach basische Anionenaustauschermasse besitzen bevorzugt eine innere Oberfläche (gemessen nach Methode BET) von mehr als 25 m²/g, vorzugsweise 50 bis 100 m²/g.

### Ausführungsbeispiel 1

In eine Lösung von 1,104 kg 99,5%iges Glycerin (12 mol) und 31,5 g 35%ige Essigsäure wurden bei ca. 383 K 2,5 h lang Chlorwasserstoff eingeleitet, wobei die Gewichtszunahme ca. 40 % betrug. 500 g dieser Lösung wurden unter Rühren innerhalb von 1,25 h in einen Reaktor eingetragen, worin sich 92 g (1 mol) Glycerin, 269,4 g 50%ige Natronlauge und 160 ml destilliertes Wasser bei 393 K befanden.

Nach weiteren 5 h wurde das Reaktionsgemisch mit destilliertem Wasser verdünnt, über Ionenaustauscher entsalzt und abschließend im Vakuum eingedampft.

Das Produktgemisch hatte folgende Zusammensetzung (in Gewichtsprozent):
Glycerin 29,1, cylisches Diglycerin 1,2, Diglycerin 31,7, cyclisches Triglycerin 1,7, Triglycerin 17,1, cyclisches Tetraglycerin 0,9, Tetraglycerin 9,1, cyclisches Pentaglycerin 0,1, Pentaglycerin 4,9, Hexaglycerin 2,8, Heptaglycerin 1,3.

### Ausführungsbeispiel 2

111 g (1 mol) α-Monochlorhydrin wurden unter Rühren in einen Reaktor eingetragen, worin sich 92 g (1 mol) Glycerin und 81 g 50%ige Natronlauge (1,1 mol) bei 343 - 358 K befanden. Nach 5 min wurde das Reaktionsgemisch mit destilliertem Wasser verdünnt, über Ionenaustauscher entsalzt und abschließend im Vakuum eingedampft. Das Produktgemisch hatte folgende Zusammensetzung (in Gew.-%):
Glycerin 35,0, cyclisches Diglycerin 0,3, Diglycerin 33,5, cyclisches Triglycerin 0,2, Triglycerin 18,0, cyclisches Tetraglycerin 0,1, Tetraglycerin 8,4, Pentaglycerin 3,4, Hexaglycerin 1,2, Heptaglycerin 0,1.

### Ausführungsbeispiel 3

111 g (1 mol) α-Monochlorhydrin wurden unter Rühren in einen Reaktor eingetragen, worin sich 166 g (1 mol) Diglycerin und 88 g 55%ige Natronlauge (1,1 mol) bei 343-358 K befanden. Nach 5 min. wurde das Reaktionsgemisch mit destilliertem Wasser verdünnt, über Ionenaustauscher entsalzt und abschließend im Vakuum eingedampft.

Das Produktgemisch hatte folgende Zusammensetzung (in Gew.-%):
Glycerin 4,5, cyclisches Diglycerin 0,1, Diglycerin 35,4, cyclisches Triglycerin 0,2, Triglycerin 30,8, cyclisches Tetraglycerin 0,1, Tetraglycerin 17,5, Pentaglycerin 8,0, Hexaglycerin 3,0, Heptaglycerin 0,4.

### Ausführungsbeispiel für das Verfahren unter Verwendung von Ionenaustauschern:

Das nach dem erfindungsgemäßen Verfahren hergestellte Polyglycerin und/oder Diglycerin wird mit Wasser auf eine 55 gew.-%ige Polyglycerinlösung, vorzugsweise Diglycerinlösung, verdünnt.

Die erhaltene Lösung wird bei einer Temperatur zwischen 40 bis 60°C nacheinander durch einen stark sauren Ionenaustauscher mit einer Ionenaustauschermasse, die über einen Sieb- bzw. Düsenboden angeordnet ist, geleitet.

Die den Siebboden verlassende polyglycerinhaltige, vorzugsweise diglycerinhaltige Lösung tritt in den nachgeschalteten Ionenaustauschbehälter ein, in welchem über dem Düsen- bzw. Siebboden eine schwach basische Ionenaustauschermasse angeordnet ist, die von einer inerten Preßmasse überlagert wird. Die aus den Anionenaustauscher austretende wäßrige Lösung mit einem Salzgehalt von weniger als 0,5 Gew.-% wird zur Destillationsanlage überführt und durch fraktionierte Destillation aufgetrennt.

## Patentansprüche

1. Verfahren zur Herstellung von Polyglycerinen durch Umsetzung von Glycerinen in der Wärme dadurch gekennzeichnet, daß das Glycerin und/oder Diglycerin bei Temperaturen von 340 K bis 410 K mit gasförmigem Chlorwasserstoff oder mit konzentrierter Salzsäure in Gegenwart eines Katalysators umgesetzt und das entstandene Gemisch oder das aus dem Gemisch abgetrennte α-Monochlorhydrin des Glycerins oder Diglycerins bei Temperaturen von 320 K bis 410 K dem vorgelegten Alkaliglycerinolat und/oder Alkalidiglycerinolat zugegeben wird, das zusätzlich eine alkalisch reagierende Substanz enthält, wonach das so hergestellte Glycerin und/oder Diglycerin-Polyglyceringemisch durch Sedimentation und/oder Zentrifugation von dem entstandenen Alkalichlorid getrennt, das Glycerin- und/oder Diglycerin- Polyglyceringemisch durch Wasserzugabe auf eine 70 bis 40 Gew.-%ige Lösung verdünnt und bei Temperaturen von 30 bis 80 °C über eine Kombination von stark sauren Kationen- und nachfolgenden schwach basischen Anionenaustauschern geleitet und entsalzt sowie nachfolgend das nicht umgesetzte Glycerin und gegebenenfalls ein Teil des Diglycerins durch fraktionierte Destillation von dem Polyglycerin, das arm an cyclischen Komponenten ist, getrennt wird.

2. Verfahren nach Anspruch 1, dadurch gekennzeichnet, daß die Umsetzung des Glycerins und/oder Diglycerins mit gasförmigem Chlorwasserstoff oder mit konzentrierter Salzsäure in Gegenwart einer flüssigen gesättigten C₁-C₃ Carbonsäure, in einer Konzentration des Katalysators (bezogen auf die Gesamtgewichtsmenge des eingesetzten Glycerins und/oder Diglycerins) von
0,05 bis 3 Gew.-%, vorzugsweise
0,5 bis 2 Gew.-%
erfolgt.

3. Verfahren nach Ansprüchen 1 und 2, dadurch gekennzeichnet, daß das mit gasförmigem Chlorwasserstoff oder mit konzentrierter Salzsäure umzusetzende Glycerin und/oder Diglycerin einen Glycerin- und/ oder Diglyceringehalt von
mehr als 80 Gew.-%, vorzugsweise
mehr als 90 Gew.-%,
aufweist.

4. Verfahren nach einem oder mehreren der Ansprüche 1 bis 3, dadurch gekennzeichnet, daß das eingesetzte Alkaliglycerinolat und/oder Alkalidiglycerinolat einen Alkaliglycerinolat- oder Alkalidiglycerinolatgehalt von
mehr als 80 Gew.-%, vorzugsweise
mehr als 90 Gew.-%
aufweist und zusätzlich einen alkalisch reagierenden Bestandteil, vorzugsweise Alkalihydroxid, enthält.

5. Verfahren nach einem oder mehreren der Ansprüche 1 bis 4, dadurch gekennzeichnet, daß als Chlorwasserstoffgas reiner Chlorwasserstoff oder ein Chlorwasserstoffgasgemisch, das bei der Vinylchloridherstellung und/oder Allylchloridherstellung anfällt, eingesetzt wird.

6. Verfahren nach einem oder mehreren der Ansprüche 1 bis 5, dadurch gekennzeichnet, daß als konzentrierte Salzsäure eine Salzsäure eingesetzt wird, die ein Gehalt von
10 bis 40 Gew.-%, vorzugsweise
30 bis 35 Gew.-%,
HCl aufweist.

7. Verfahren nach einem oder mehreren der Ansprüche 1 bis 6, dadurch gekennzeichnet, daß das α-Monochlorhydrin des Glycerins und/oder Diglycerins in das vorgelegte Glycerinolat und/oder Diglycerinolat unter Umrühren zugefügt wird, vorzugsweise durch kontinuierliche Zugabe.

8. Verfahren nach einem oder mehreren der Ansprüche 1 bis 7, dadurch gekennzeichnet, daß das α-Monochlorhydrin des Glycerins und/oder Diglycerins gleichzeitig mit dem Glycerinolat und/oder Diglycerinolat in einen Durchlaufreaktor eingetragen werden.

9. Verfahren nach einem oder mehreren der Ansprüche 1 bis 8, dadurch gekennzeichnet, daß das Glycerin und/oder Diglycerin-Polyglyceringemisch durch Wasserzugabe auf eine 60 bis 50 gew.-%ige Lösung verdünnt und bei Temperaturen von 40 bis 60 °C über eine Kombination von stark sauren Kationen- und nachfolgenden schwach basischen Anionenaustauschern entsalzt wird.

10. Verfahren nach einem oder mehreren der Ansprüche 1 bis 9, dadurch gekennzeichnet, daß die Regenerierung der Kationenaustauschermasse in den Kationenaustauschern mittels einer Gleichstrom- oder Verbund-Gleichstrom-Regenerierung erfolgt.

11. Verfahren nach einem oder mehreren der Ansprüche 1 bis 10, dadurch gekennzeichnet, daß nach der Regenerierung die Salze ausgewaschen, nach Beendigung des Auswaschvorganges die polyglycerinhaltige, vorzugsweise diglycerinhaltige Lösung durch die Ionenaustauscher durchgeleitet wird und die den Anionenaustauscher verlassende polyglycerinhaltige, vorzugsweise diglycerinhaltige Lösung bis zur Erreichung eines Polyglyceringehaltes, vorzugsweise Diglyceringehaltes von 20 Gew.-%, vorzugsweise bis zur Erreichung eines Polyglyceringehaltes, vorzugsweise Diglyceringehaltes von 15 Gew.-% zurückgeleitet und zur Herstellung der
70 - 40 gew.-%igen, vorzugsweise
60 - 50 gew.-%igen,
polyglycerinhaltigen, vorzugsweise diglycerinhaltigen Ausgangslösung mitverwendet wird.

12. Verfahren nach einem oder mehreren der Ansprüche 1 bis 11, dadurch gekennzeichnet, daß das die Durchleitung der polyglycerinhaltigen, vorzugweise diglycerinhaltigen Lösung durch die Ionenaustauscher unter Überdruck erfolgt.

13. Verfahren nach einem oder mehreren der Ansprüche 1 bis 12, dadurch gekennzeichnet, daß die polyglycerinhaltige, vorzugsweise diglycerinhaltige Lösung unter einem Druck von
1,1 - 10 bar, vorzugsweise
2 - 6 bar,
durch einen oder mehrere Kationenaustauscher und mindestens einen Anionenaustauscher geleitet wird.

14. Verfahren nach einem oder mehreren der Ansprüche 1 bis 13, dadurch gekennzeichnet, daß die Ionenaustauschermasse des Kationenaustauschers und/oder Anionenaustauschers von einer Siebplatte, Lockplatte oder einer in der Höhenrichtung des Ionenaustauschers verschiebbar angeordneten, die Austauschermasse abdeckenden und einen gleichmäßigen Flüssigkeitsdurchtritt ermöglichenden Vorrichtung und/oder einer inerten Preßmasse und/oder elastischen Kunststoffmasse bedeckt ist.

15. Verfahren nach einem oder mehreren der Ansprüche 1 bis 14, dadurch gekennzeichnet, daß die eingesetzten Kationenaustauschermassen und Anionenaustauschermassen temperaturbeständig
bis über 80 °C, vorzugsweise
bis über 100 °C,
sind.

16. Verfahren nach einem oder mehreren der Ansprüche 1 bis 15, dadurch gekennzeichnet, daß die stark saure Kationenaustauschermasse und die schwach basische Anionenaustauschermasse eine innere Oberfläche (gemessen nach Methode BET) von
mehr als 25 m², vorzugsweise
50 bis 100 m²,
aufweisen.

17. Verfahren nach einem oder mehreren der Ansprüche 1 bis 16, dadurch gekennzeichnet, daß die polyglycerinhaltige, vorzugsweise diglycerinhaltige Lösung mit einer Strömungsgeschwindigkeit von
0,5 m/h bis 15 m/h, vorzugsweise
1 m/h bis 5 m/h,
durch die Ionenaustauscher geleitet wird.

## Claims

1. A process for the preparation of polyglycerines by reacting glycerines under heat, characterized in that the glycerine and/or diglycerine is reacted at temperatures of from 340 K to 410 K with gaseous hydrogen chloride or with concentrated hydrochloric acid in the presence of a catalyst and the mixture obtained or the α-monochlorohydrin of the glycerine or diglycerine separated from the mixture is added at temperatures of from 320 K to 410 K to the alkali glycerinolate and/or alkali diglycerinolate provided, which additionally contains an alkalinely reacting substance, whereafter the glycerine and/or diglycerine/polyglycerine mixture prepared in this way is separated by sedimentation and/or centrifuging from the alkaline chloride arising, the glycerine- and/or diglycerine-polyglycerine mixture is diluted to a 70 to 40 % by weight solution by the addition of water and is conveyed at temperatures of from 30 to 80°C over a combination of highly acidic cation and subsequent weakly alkaline anion exchangers and desalinated, the unconverted glycerine and optionally part of the diglycerine subsequently being separated from the polyglycerine, which is low in cyclic components, by fractional distillation.

2. A process according to claim 1, characterized in that the reaction of the glycerine and/or diglycerine with gaseous hydrogen chloride or with concentrated hydrochloric acid in the presence of a liquid, saturated C₁-C₃ carboxylic acid, is effected in a catalyst concentration (with respect to the total weight of the glycerine and/or diglycerine used) of from
0.05 to 3 % by weight, preferably
0.5 to 2 % by weight.

3. A process according to claims 1 and 2, characterized in that the glycerine and/or diglycerine to be reacted with gaseous hydrogen chloride or with concentrated hydrochloric acid has a glycerine and/or diglycerine content of
more than 80 % by weight, preferably
more than 90 % by weight.

4. A process according to any one of claims 1 to 3, characterized in that the alkali glycerinolate and/or alkali diglycerinolate used has an alkali glycerinolate or alkali diglycerinolate content of
more than 80 % by weight, preferably
more than 90 % by weight
and additionally contains an alkalinely reacting component, preferably alkali hydroxide.

5. A process according to any one of claims 1 to 4, characterized in that pure hydrogen chloride or a hydrochloric acid gas mixture, which is obtained during vinyl chloride and/or allyl chloride production, is used as the hydrochloric acid gas.

6. A process according to any one of claims 1 to 5, characterized in that a hydrochloric acid with a content of from
10 to 40 % by weight, preferably
30 to 35 % by weight
HCl is used as the concentrated hydrochloric acid.

7. A process according to any one of claims 1 to 6, characterized in that the α-monochlorohydrin of the glycerine and/or diglycerine is added to the glycerinolate and/or diglycerinolate provided with stirring, preferably by continuous addition.

8. A process according to any one of claims 1 to 7, characterized in that the α-monochlorohydrin of the glycerine and/or diglycerine are introduced into a continuous reactor at the same time as the glycerinolate and/or diglycerinolate.

9. A process according to any one of claims 1 to 8, characterized in that the glycerine- and/or diglycerinepolyglycerine mixture is diluted by the addition of water to a 60 to 50 % by weight solution and is desalinated at temperatures of from 40 to 60°C by means of a combination of strongly acidic cation and subsequent weakly alkaline anion exchangers.

10. A process according to any one of claims 1 to 9, characterized in that regeneration of the cation exchange medium in the cation exchangers is effected by means of co-current or compounded co-current regeneration.

11. A process according to any one of claims 1 to 10, characterized in that after regeneration the salts are rinsed, after completion of the rinsing process the polyglycerine-containing, preferably diglycerine-containing solution is conveyed through the ion exchanger and the polyglycerine-containing, preferably diglycerine-containing solution leaving the anion exchanger is conveyed back to reach a polyglycerine, preferably diglycerine, content of 20 % by weight, preferably to reach a polyglycerine, preferably diglycerine, content of 15 % by weight, and is co-used to produce the
70 - 40 % by weight, preferably
60 - 50 % by weight
polyglycerine-containing, preferably diglycerine-containing, starting solution.

12. A process according to any one of claims 1 to 11, characterized in that the conveying of the polyglycerine-containing, preferably diglycerine-containing, solution through the ion exchanger is effected under overpressure.

13. A process according to any one of claims 1 to 12, characterized in that the polyglycerine-containing, preferably diglycerine-containing solution is conveyed through one or several cation exchangers and at least one anion exchanger under a pressure of from
1.1 - 10 bar, preferably
2 - 6 bar.

14. A process according to any one of claims 1 to 13, characterized in that the ion exchange medium of the cation exchanger and/or anion exchanger is covered by a sieve plate, perforated plate or a device which is arranged to be displaceable in the vertical direction of the ion exchanger, covers the exchange medium and enables even flow-through and/or by an inert moulded compound and/or resilient plastics compound.

15. A process according to any one of claims 1 to 14, characterized in that the cation exchange media and anion exchange media used are resistant to temperatures of
over 80°C, preferably
over 100°C.

16. A process according to any one of claims 1 to 15, characterized in that the strongly acidic cation exchange medium and the weakly alkaline anion exchange medium comprise an inner surface area (measured by the BET method) of
more than 25 m², preferably
50 to 100 m².

17. A process according to any one of claims 1 to 16, characterized in that the polyglycerine-containing, preferably diglycerine-containing solution is conveyed through the ion exchanger at a flow speed of from
0.5 m/h to 15 m/h, preferably
1 m/h to 5 m/h.

## Revendications

1. Procédé de préparation de polyglycérines par décomposition de glycérines à chaud, caractérisé en ce qu'on décompose de la glycérine et/ou de la diglycérine à des températures de 340 à 410°K avec du gaz chlorhydrique ou de l'acide chlorhydrique concentré en présence d'un catalyseur en un mélange constitué en substance d'alpha-monochlorhydrine de la glycérine ou de la diglycérine et de glycérine et/ou de diglycérine et on ajoute le mélange obtenu ou l'alpha-monochlorhydrine de la glycérine ou de la diglycérine séparée du mélange à des températures de 320 à 410°K à du glycérinolate alcalin et/ou du diglycérinolate alcalin, après quoi on sépare le mélange de glycérine et/ou de diglycérine et de polyglycérines des chlorures alcalins obtenus, on dilue le mélange de glycérine et/ou de diglycérine et de polyglycérines par addition d'eau pour obtenir une solution à 70 à 40 % en poids, on le fait passer à des températures de 30 à 80°C sur une combinaison d'échangeurs de cations fortement acides suivis d'échangeurs d'anions faiblement basiques pour le déminéraliser, puis on sépare la glycérine non décomposée et éventuellement une partie de la diglycérine par fraction distillée de la polyglycérine qui est pauvre en composants cycliques.

2. Procédé selon la revendication 1, caractérisé en ce que la décomposition de la glycérine et/ou de la diglycérine par du gaz chlorhydrique ou par de l'acide chlorhydrique concentré se fait en l'occurrence de préférence en présence d'un acide carboxylique en C₁-C₃ liquide insaturé et d'une concentration du catalyseur (par rapport à la quantité totale en poids de la glycérine et/ou de la diglycérine utilisées) de
0,05 à 3 % en poids, de préférence
0,5 à 2 % en poids.

3. Procédé selon les revendications 1 et 2, caractérisé en ce que la glycérine et/ou la diglycérine à décomposer par le gaz chlorhydrique ou l'acide chlorhydrique concentré présentent une teneur en glycérine et/ou en diglycérine de
plus de 80 % en poids, de préférence
plus de 90 % en poids.

4. Procédé selon une ou plusieurs des revendications 1 à 3, caractérisé en ce que le glycérinolate alcalin et/ou le diglycérinolate alcalin utilisés présentent une teneur en glycérinolate allcalin ou en diglycérinolate alcalin
de plus de 80 % en poids, de préférence
de plus de 90 % en poids
et contiennent de préférence en plus un composant à réaction alcaline, de préférence un hydroxyde alcalin.

5. Procédé selon une ou plusieurs des revendications 1 à 4, caractérisé en ce qu'on utilise comme gaz chlorhydrique dans le cadre du procédé de l'invention du gaz chlorhydrique pur ou un mélange de gaz chlorhydrique, de préférence un mélange de gaz chlorhydrique qui résulte de la fabrication de chlorure de vinyle et/ou de celle du chlorure d'allyle.

6. Procédé selon une ou plusieurs des revendications 1 à 5, caractérisé en ce qu'on utilise à titre d'acide chlorhydrique concentré un acide chlorhydrique qui présente une teneur en HCl de
10 à 40 % en poids, de préférence
30 à 35 % en poids.

7. Procédé selon une ou plusieurs des revendications 1 à 6, caractérisé en ce qu'on ajoute l'alpha-monochlorhydrine de la glycérine et/ou de la diglycérine au glycérinolate et/ou au diglycérinolate précités sous agitation, de préférence par addition en continu.

8. Procédé selon une ou plusieurs des revendications 1 à 7, caractérisé en ce qu'on fait passer l'alpha-monochlorhydrine de la glycérine et/ou de la diglycérine simultanément avec le glycérinolate et/ou le diglycérinolate dans un réacteur de passage.

9. Procédé selon une ou plusieurs des revendications 1 à 8, caractérisé en ce qu'on dilue le mélange de glycérine et/ou de diglycérine et de polyglycérines obtenu par addition d'eau pour obtenir une solution à 60 à 50 % en poids, qui est déminéralisée à des températures de 40 à 60°C, par passage sur une combinaison d'échangeurs de cations fortement acides et ensuite d'échangeurs d'anions faiblement basiques.

10. Procédé selon une ou plusieurs des revendications 1 à 9, caractérisé en ce que la régénération de la matière d'échange cationique des échangeurs de cations se fait de préférence par régénération à co-courant ou à co-courant mixte.

11. Procédé selon une ou plusieurs des revendications 1 à 10, caractérisé en ce que, après la régénération, les sels sont séparés par lavage, à la fin du processus de lavage, la solution à teneur en polyglycérines, de préférence en diglycérine est acheminée à travers les échangeurs d'ions et la solution à teneur en polyglycérines, de préférence en diglycérine quittant les échangeurs d'anions est repassée en sorte d'atteindre une teneur en polyglycérines, de préférence en diglycérine de 20 % en poids, de préférence en sorte d'atteindre une teneur en polyglycérines, de préférence en diglycérine de 15 % en poids et est utilisée conjointement pour préparer la solution de départ à teneur en polyglycérines, de préférence en diglycérine de
70 à 40 % en poids, de préférence
60 à 50 % en poids.

12. Procédé selon une ou plusieurs des revendications 1 à 11, caractérisé en ce que l'acheminement de la solution à teneur en polyglycérines, de préférence en diglycérine à travers les échangeurs d'ions se fait de préférence sous pression.

13. Procédé selon une ou plusieurs des revendications 1 à 12, caractérisé en ce que la solution à teneur en polyglycérines, de préférence en diglycérine est acheminée sous une pression de
1,1 à 10 bars, de préférence
2 à 6 bars,
à travers un ou plusieurs échangeurs de cations et au moins un échangeur d'anions.

14. Procédé selon une ou plusieurs des revendications 1 à 13, caractérisé en ce que la matière échangeuse d'ions de l'échangeur de cations et/ou de l'échangeur d'anions est recouverte de préférence d'un tamis, d'une plaque perforée ou d'un dispositif agencé de manière à pouvoir bouger dans le sens de la hauteur de l'échangeur d'ions, recouvrant la matière échangeuse d'ions et permettant un passage uniforme du liquide, et/ou d'une matière moulée inerte et/ou d'une masse de matière plastique élastique.

15. Procédé selon une ou plusieurs des revendications 1 à 14, caractérisé en ce que les matières échangeuses de cations et d'anions utilisées résistent à des températures
jusqu'à plus de 80°C, de préférence
jusqu'à plus de 100°C.

16. Procédé selon une ou plusieurs des revendications 1 à 15, caractérisé en ce que la matière échangeuse de cations fortement acide et la matière échangeuse d'anions faiblement basique possèdent de préférence une surface interne (mesurée selon la méthode BET) de
plus de 25 m², de préférence
50 à 100 m².

17. Procédé selon une ou plusieurs des revendications 1 à 16, caractérisé en ce que la solution à teneur en polyglycérines, de préférence en diglycérine est acheminée à travers les échangeurs d'ions à une vitesse d'écoulement de
0,5 à 15 m/h, de préférence
1 à 5 m/h.
